(19) Europäisches Patentamt
European Patent Office
Office européen des brevets

(11) **EP 4 056 170 A1**

(12) **EUROPEAN PATENT APPLICATION**
published in accordance with Art. 153(4) EPC

(43) Date of publication:
**14.09.2022  Bulletin 2022/37**

(21) Application number: **20883993.6**

(22) Date of filing: **08.11.2020**

(51) International Patent Classification (IPC):
*A61K 9/16* (2006.01)        *A61K 9/00* (2006.01)
*A61P 25/28* (2006.01)

(86) International application number:
**PCT/KR2020/015565**

(87) International publication number:
**WO 2021/091333 (14.05.2021 Gazette 2021/19)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **08.11.2019  KR 20190142635**

(71) Applicant: **Whanin Pharmaceutical. Co., Ltd.
Seoul 05855 (KR)**

(72) Inventors:
  • **KIM, Byung Hyuck
    Suwon-si Gyeonggi-do 16459 (KR)**
  • **JUNG, Chan Eun
    Suwon-si Gyeonggi-do 16509 (KR)**

  • **HONG, Yong Soon
    Suwon-si Gyeonggi-do 16528 (KR)**
  • **SHIN, Ho Chul
    Seongnam-si Gyeonggi-do 13600 (KR)**
  • **CHOI, Dong Hoon
    Suwon-si Gyeonggi-do 16694 (KR)**
  • **KIM, Seo Yeon
    Seoul 06269 (KR)**
  • **CHA, Se Rom
    Yongin-si Gyeonggi-do 16840 (KR)**
  • **RYU, Min Ji
    Seoul 05501 (KR)**

(74) Representative: **Louis Pöhlau Lohrentz
Patentanwälte
Postfach 30 55
90014 Nürnberg (DE)**

(54) **MICROSPHERE FOR CONTINUOUS RELEASE AND METHOD FOR MANUFACTURING SAME**

(57)    Disclosed are a microsphere capable of continuous drug release and a method for manufacturing the same.

EP 4 056 170 A1

[Fig. 15]

Blood donepezil concentration in SD rat

## Description

### Technical field

The present invention relates to a drug-containing microsphere used for sustained release injection and a method for preparing the same.

### Background art

Sustained release injections are generally manufactured by preparing microspheres in which a drug is included and putting them in an injection. Here, the microspheres are prepared to have a drug included therein. When the microsphere-containing injection is injected into the body, the drug is slowly released from the microspheres injected into the body so as to exhibit a pharmaceutical effect continuously.

Therefore, when an injection solution comprising microspheres for sustained release is injected into the body, the pharmacological effect of the drug may be exhibited for a prolonged period of time by allowing the drug to be slowly released from the microspheres in the body.

By such sustained release injection, the number of invasions into the body may be reduced, and patient medication compliance may be improved. For example, when a drug which is to be administered by oral route once or twice a day or by injection once a day is prepared into a sustained release injection containing microspheres as above, the drug may be formulated so that the medicinal effect lasts for 30 days after administration of a single injection.

Therefore, patient medication compliance may be improved by overcoming the inconvenience of having to orally take the drug every day or receive injections every day.

In particular, in the case of patients with dementia or Alzheimer's disease, Parkinson's disease, or a depressive disorder who need to take therapeutic agents or antipsychotics acting on the central nervous system but have difficulty in taking them regularly, it may be possible to provide a very large advantage in terms of patient medication compliance when a drug is formulated into a sustained release dosage form such as a microsphere-containing injection, and administered, for example, once a month.

Currently, microspheres are generally prepared by a solvent evaporation method. The solvent evaporation method is a method of dissolving a polymer material and a drug in a volatile organic solvent, and then evaporating the organic solvent so that the drug is included in the microsphere.

However, microspheres prepared in this way have a problem that the drug is rapidly released in the initial stage when injected into the body because the drug is present on the surface of the microsphere. As such, when the drug is rapidly released in the initial stage of injection, the blood concentration of the drug rises rapidly, which may cause adverse effects to the patient. In particular, when the blood concentration of a drug acting on the central nervous system rises rapidly, adverse effects such as tremor or miosis may suddenly occur.

Therefore, it is important to suppress such initial drug release from the microsphere.

In general, in order to suppress the initial drug release from the microsphere, the surface is to be washed. For example, the drug deposited on the surface of the microsphere prepared by solvent evaporation, etc. may be removed by washing the drug deposited on the surface of the microsphere with an aqueous ethanol solution, or washing with a Tween-based or Span-based surfactant.

However, when washing the surface of microsphere as above, the washing process is complicated, the process time is long, and the process of treating with ethanol or a surfactant may adversely affect the microsphere or the drug itself, and thus such process may not be preferable.

Therefore, it is necessary to develop a novel method for preparing microspheres which may suppress the initial drug release from the microsphere in a short process time with a simple process without significantly affecting the microsphere or the drug included therein.

### Detailed description of invention

### Technical task

Accordingly, the present inventors studied a method for suppressing the initial drug release from the microsphere, and as a result, have found that, when the microsphere was prepared by adding a salt to a continuous phase, it is possible to prevent the formation of drug on the surface of the microsphere and also prevent the formation of pores on the surface of the microsphere, thereby preventing the drug from being excessively released in the initial stage when injected into the body.

Therefore, it is an object of the present invention to provide a method for preparing microspheres whose initial drug release is suppressed by simplifying the process and reducing the process time without significantly affecting the microsphere or the drug included therein.

**Means for solving technical task**

The present invention relates to a method for preparing a sustained release microsphere containing a drug.

Specifically, the method for preparing a microsphere according to the present invention comprises dissolving an active ingredient and a biodegradable polymer in an organic solvent to prepare a dispersed phase; dissolving a salt in water to prepare a continuous phase; mixing and stirring the dispersed phase and the continuous phase to form an emulsion; removing the organic solvent; and drying.

According to the present invention, drug crystals are not produced around or on the surface of the microsphere, and thus it is not necessary to wash the microsphere with ethanol or a surfactant.

In the present invention, as the salt included in the continuous phase, the salt such as sodium chloride (NaCl), potassium chloride (KCl), calcium chloride ($CaCl_2$), magnesium sulfate ($MgSO_4$), sodium sulfate ($Na_2SO_4$), mannitol, ammonium, potassium sulfate, disodium phosphate, dipotassium phosphate, trisodium phosphate, disodium citrate, trisodium citrate, or sodium succinate may be used, and the salt has a concentration of 1 to 10% (w/v), preferably 2 to 8% (w/v) in the continuous phase.

The microsphere according to the present invention is injected into the body having the drug included therein, and thus a biodegradable polymer such as polylactide, poly(lactide-co-glycolide), polyglycolactide and poly(lactide-co-glycolide)glucose may be used.

In the present invention, the continuous phase may be prepared and used to further comprise a water-soluble polymer. For example, at least one selected from the group consisting of polyvinyl alcohol, polysorbate, poloxamer, polyvinylpyrrolidone, polyvinylmethyl ether, and polyvinyl ether may be used. The dispersibility of the emulsion may be maintained by the water-soluble polymer.

As an active ingredient which may be used in the present invention, a substance acting on the central nervous system is more effective in terms of patient medication compliance. As the active ingredient, a substance showing therapeutic activity for dementia such as donepezil, a therapeutic agent for Parkinson's disease such as pramipexole, rotigotine, and ropinirole, an antipsychotic such as risperidone, blonanserin, and rulasidone, or an alcoholism therapeutic drug such as naltrexone, etc. may be used.

In the microsphere of the present invention, the drug may be present in an amount of about 30-50%, preferably 40-50%, with respect to the total weight of the microsphere.

As another aspect of the present invention, the present invention relates to a microsphere prepared by the above preparation method.

As another aspect of the present invention, the present invention relates to a sustained release injection comprising the microsphere as described above.

**Effect of invention**

The injection containing sustained release microspheres according to the present invention prevents the formation of drug crystals around or on the surface of the microsphere and improves the drug entrapment efficiency into the microsphere, thereby preventing the drug from being excessively released in the initial stage when administered. In addition, since the drug is continuously released, it is possible to increase patient medication compliance and reduce adverse effects caused by dose dumping.

**Brief description of drawings**

Figs. 1 to 12 are photographs showing SEM images for confirming the morphology of donepezil microspheres according to each of the examples and comparative examples;

Figs. 13 and 14 are photographs showing enlarged SEM images of the surface of microspheres prepared in comparative example 1 and example 1, respectively; and

Fig. 15 is a graph showing the concentration in the blood obtained for 24 hours after administration of the microspheres prepared in example 1, comparative example 1, comparative example 2, and comparative example 2-1 to SD rats.

**Best mode for carrying out the invention**

Hereinafter, examples will be described in detail to help the understanding of the present invention. However, the following examples are merely illustrative of the contents of the present invention, and the scope of the present invention is not limited to the following examples. The examples of the present invention are provided to explain the present invention more completely to those of ordinary skill in the art.

Example 1: Preparation of microspheres by adding polylactide polymer to NaCl 1% (w/v) continuous phase

2 g (amount of drug loaded in microspheres (hereinafter, "drug loading") is 40%) of donepezil (manufacturer: Neuland Laboratories, India) and 3 g of poly D,L-lactide (Resomer R 203 H; manufacturer: Evonik, Germany), which is a biodegradable polymer, were added to 9 g of dichloromethane (manufacturer: Daejung Chemicals & Metals Co., Ltd., South Korea) and completely dissolved by stirring to prepare a polymer solution, which is a dispersed phase. In addition, 6.25 g of polyvinyl alcohol and 12.5 g of NaCl were dissolved in 1.25 L of water to prepare a continuous phase.

After putting the continuous phase in a double-jacket beaker and maintaining the temperature at 10°C or below using a constant temperature circulating water bath, the dispersed phase (i.e., donepezil-containing polymer solution) was added to the continuous phase (i.e., polyvinyl alcohol and NaCl aqueous solution) and stirred at high speed to form an emulsion.

Then, in order to remove the organic solvent and obtain solidified microspheres, the organic solvent was volatilized at a temperature of 47°C for 2 hours, and then slowly cooled to 10°C for 1 hour. After washing the hardened microspheres with water for injection several times, and going through wet filtration using a sieve and freeze-drying, donepezil-containing microspheres were finally obtained.

Example 1-1: Preparation of microspheres by adding polylactide polymer to NaCl 1% (w/v) continuous phase

2.25 g (drug loading: 45%) of donepezil (manufacturer: Neuland Laboratories, India) and 2.75 g of poly D,L-lactide (Resomer R 203 H; manufacturer: Evonik, Germany), which is a biodegradable polymer, were added to 8.25 g of dichloromethane (manufacturer: Daejung Chemicals & Metals Co., Ltd., South Korea) and completely dissolved by stirring to prepare a polymer solution, which is a dispersed phase. In addition, 6.25 g of polyvinyl alcohol and 12.5 g of NaCl were dissolved in 1.25 L of water to prepare a NaCl 1% (w/v) continuous phase. The rest of the preparation method was carried out in the same manner as in example 1 to prepare microspheres.

Example 2: Preparation of microspheres by adding polylactide polymer to NaCl 5% (w/v) continuous phase

Except that NaCl was added to be 5% (w/v) while preparing the continuous phase, the rest of the preparation method was carried out in the same manner as in example 1 to prepare microspheres.

Example 2-1: Preparation of microspheres by adding polylactide polymer to NaCl 10% (w/v) continuous phase

Except that NaCl was added to be 10% (w/v) while preparing the continuous phase, the rest of the preparation method was carried out in the same manner as in example 1 to prepare microspheres.

Example 3: Preparation of microspheres by adding polylactide polymer to KCl 1% (w/v) continuous phase

Except that KCl was added instead of NaCl to be 1% (w/v) while preparing the continuous phase, the rest of the preparation method was carried out in the same manner as in example 1 to prepare microspheres.

Example 3-1: Preparation of microspheres by adding polylactide polymer to KCl 5% (w/v) continuous phase

Except that KCl was added instead of NaCl to be 5% (w/v) while preparing the continuous phase, the rest of the preparation method was carried out in the same manner as in example 1 to prepare microspheres.

Example 4: Preparation of microspheres by adding poly(D,L-lactide-co-glycolide) polymer to NaCl 5% (w/v) continuous phase

2 g (drug loading: 40%) of donepezil (manufacturer: Neuland Laboratories, India) and 3 g of poly D,L-lactide-co-glycolide (PLGA; Resomer RG 753 H; manufacturer: Evonik, Germany), which is a biodegradable polymer, were added to 9 g of dichloromethane (manufacturer: Daejung Chemicals & Metals Co., Ltd., South Korea) and completely dissolved by stirring to prepare a polymer solution, which is a dispersed phase. The rest of the preparation method was carried out in the same manner as in example 1, except that NaCl was added to be 5% by weight while preparing the continuous phase, to prepare microspheres.

Comparative Example 1: Preparation of microspheres by adding polylactide polymer to a continuous phase to which no salt is added

2 g (drug loading: 40%) of donepezil (manufacturer: Neuland Laboratories, India) and 3 g of poly D,L-lactide (Resomer R 203 H; manufacturer: Evonik, Germany), which is a biodegradable polymer, were added to 9 g of dichloromethane (manufacturer: Daejung Chemicals & Metals Co., Ltd., South Korea) and completely dissolved by stirring to prepare a polymer solution, which is a dispersed phase. In addition, 6.25 g of polyvinyl alcohol was dissolved in 1.25 L of water to prepare a continuous phase (salts such as NaCl or KCl were not added).

After putting the continuous phase in a double-jacket beaker and maintaining the temperature at 10°C or below using a constant temperature circulating water bath, the dispersed phase (i.e., donepezil-containing polymer solution) was added to the continuous phase (i.e., polyvinyl alcohol aqueous solution) and stirred at high speed to form an emulsion.

Then, in order to remove the organic solvent and obtain solidified microspheres, the organic solvent was volatilized at a temperature of 47°C for 2 hours, and then slowly cooled to 10°C for 1 hour. After washing the hardened microspheres with water for injection several times, and going through wet filtration using a sieve and freeze-drying, donepezil-containing microspheres were finally obtained.

Comparative Example 1-1: Preparation of microspheres by adding polylactide polymer to a continuous phase to which no salt is added

2.25 g (drug loading: 45%) of donepezil (manufacturer: Neuland Laboratories, India) and 2.75 g of poly D,L-lactide (Resomer R 203 H; manufacturer: Evonik, Germany), which is a biodegradable polymer, were added to 8.25 g of dichloromethane (manufacturer: Daejung Chemicals & Metals Co., Ltd., South Korea) and completely dissolved by stirring to prepare a polymer solution, which is a dispersed phase. The rest of the preparation method was carried out in the same manner as in comparative example 1 to prepare microspheres.

Comparative Example 2: Preparation of microspheres using a continuous phase to which no salt is added, followed by washing with EtOH aqueous solution

2 g of donepezil (manufacturer: Neuland Laboratories, India) and 3 g of poly D,L-lactide (Resomer R 203 H; manufacturer: Evonik, Germany), which is a biodegradable polymer, were added to 9 g of dichloromethane (manufacturer: Daejung Chemicals & Metals Co., Ltd., South Korea) and completely dissolved by stirring to prepare a polymer solution, which is a dispersed phase. In addition, 6.25 g of polyvinyl alcohol was dissolved in 1.25 L of water to prepare a continuous phase (salts such as NaCl or KCl were not added).

After putting the continuous phase in a double-jacket beaker and maintaining the temperature at 10°C or below using a constant temperature circulating water bath, the dispersed phase (i.e., donepezil-containing polymer solution) was added to the continuous phase (i.e., polyvinyl alcohol aqueous solution) and stirred at high speed to form an emulsion.

Then, in order to remove the organic solvent and obtain solidified microspheres, the organic solvent was volatilized at a temperature of 47°C for 2 hours, and then slowly cooled to 10°C for 1 hour. After washing the hardened microspheres with water for injection several times, and going through first wet filtration using a sieve, microspheres were primarily obtained.

After washing the primarily obtained microspheres with 20% EtOH aqueous solution at 10°C for 1 hour, and going through wet filtration again using a sieve and freeze-drying, donepezil-containing microspheres were finally obtained.

Comparative Example 2-1: Preparation of microspheres using a continuous phase to which no salt is added, followed by washing with Tween aqueous solution

The preparation method was carried out in the same manner as in comparative example 2 until primarily obtaining donepezil microspheres.

Then, after washing the primarily obtained microspheres with 3% Tween 20 aqueous solution at 10°C for 1 hour, and going through wet filtration again using a sieve and freeze-drying, donepezil-containing microspheres were finally obtained.

Comparative Example 3: Preparation of microspheres by adding poly(D,L-lactide-co-glycolide) polymer to a continuous phase to which no salt is added

2 g (theoretical drug loading: 40%) of donepezil (manufacturer: Neuland Laboratories, India) and 3 g of poly(D, L-lactide-co-glycolide) (Resomer RG 753 H; manufacturer: Evonik, Germany), which is a biodegradable polymer, were added to 9 g of dichloromethane (manufacturer: Daejung Chemicals & Metals Co., Ltd., South Korea) and completely dissolved by stirring to prepare a polymer solution, which is a dispersed phase. In addition, 6.25 g of polyvinyl alcohol was dissolved in 1.25 L of water to prepare a continuous phase (salts such as NaCl or KCl were not added).

After putting the continuous phase in a double-jacket beaker and maintaining the temperature at 10°C or below using a constant temperature circulating water bath, the dispersed phase (i.e., donepezil-containing polymer solution) was added to the polyvinyl alcohol aqueous solution (continuous phase) and stirred at high speed to form an emulsion.

Then, in order to remove the organic solvent and obtain solidified microspheres, the organic solvent was volatilized at a temperature of 47°C for 2 hours, and then slowly cooled to 10°C for 1 hour. After washing the hardened microspheres with water for injection several times, and going through wet filtration using a sieve and freeze-drying, donepezil-containing microspheres were finally obtained.

Experimental Example 1: Observation of microsphere morphology using SEM

About 20 mg of microspheres obtained in each of the examples and comparative examples was fixed to an aluminum stub using carbon tape, and coated with platinum under a vacuum level of 0.1 torr and high voltage (10 kV) for 3 minutes. Then, the microspheres were mounted on the main body (SEM stage) of SEM (equipment name: SEC-SNE 4500M Plus A, South Korea) to observe the surface morphology of the microspheres using an image analysis program (mini-SEM).

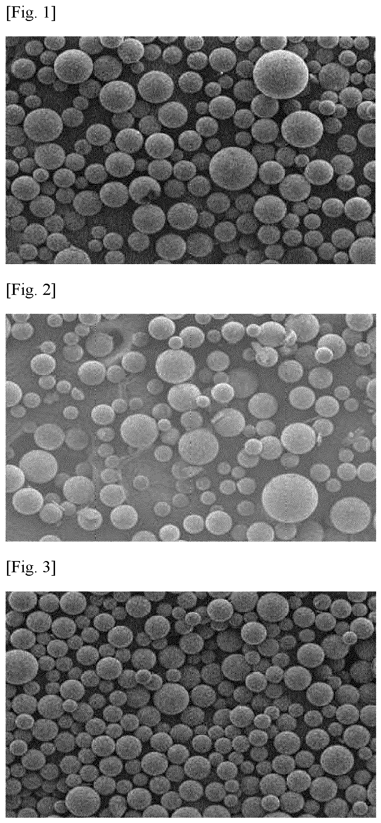

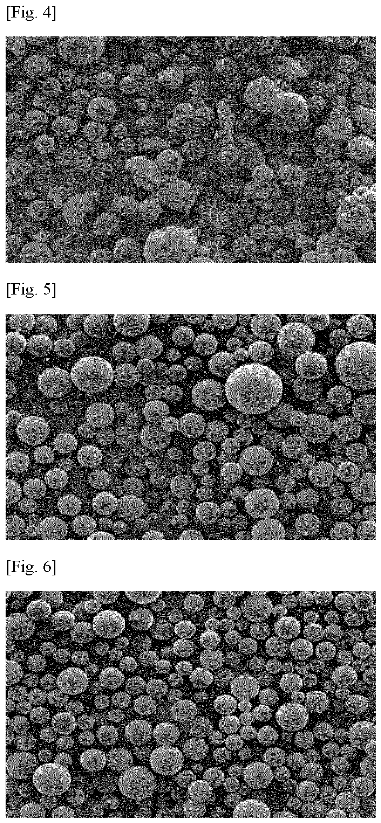

Figs. 1 to 14 are SEM images of the microspheres prepared in examples 1, 1-1, 2, 2-1, 3, 3-1 and 4, and comparative examples 1, 1-1, 2, 2-1 and 3, respectively.

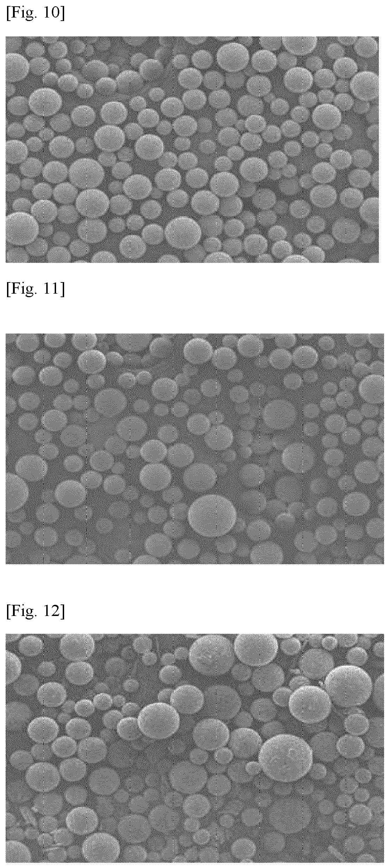

As can be seen in Figs. 1 to 7, most of the microspheres prepared in the examples did not show drug crystals on the periphery. In addition, as can be seen in Figs. 10 and 11, the microspheres prepared in comparative example 2 and comparative example 2-1 to which the conventional drug crystal removing process (i.e., ethanol washing process or surfactant washing process) was applied did not show drug crystals on the periphery.

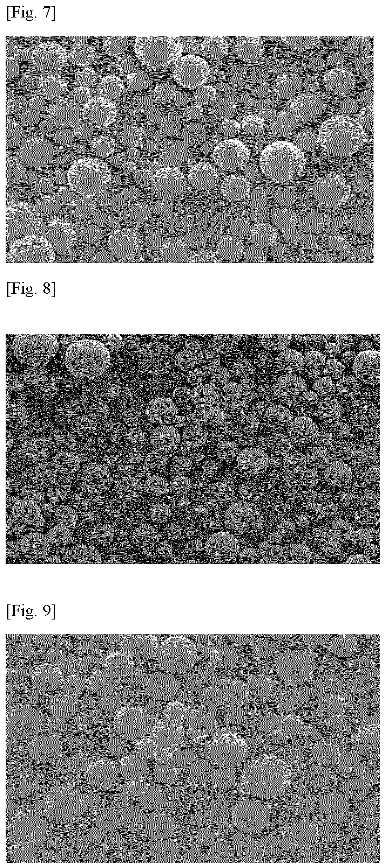

However, as can be seen in Figs. 8, 9 and 12, a large amount of drug crystals were seen on the periphery of the microspheres prepared in comparative example 1, comparative example 1-1, and comparative example 3.

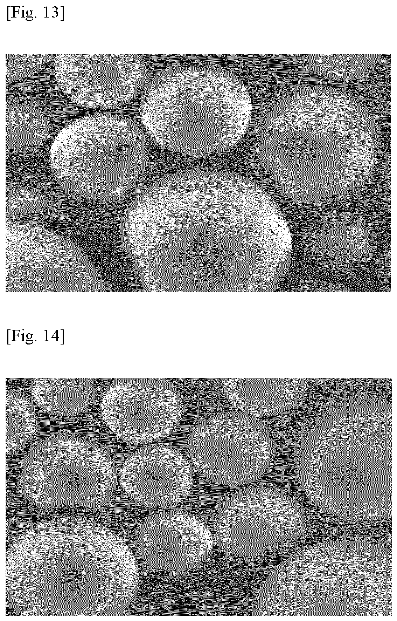

As can be seen in Fig. 13, pores were observed to be formed on the surface of the microspheres prepared in comparative example 1, but as can be seen in Fig. 14, no pores were formed on the surface of the microspheres in example 1 prepared by adding salt to the continuous phase according to the present invention.

Experimental Example 2: Removal of drug crystals from the surface of microspheres

Although the presence of drug crystals was roughly confirmed from the SEM photograph images, in order to quantitatively confirm the amount of drug crystals actually generated outside the microspheres without being included therein while preparing microspheres, the microspheres prepared in each of the examples and comparative examples 1, 1-1 and 3 were washed with 20% EtOH aqueous solution at 10°C for 1 hour and freeze-dried to remove drug crystals from the surface and the periphery of the microspheres.

Experimental Example 3: Measurement of actual content of donepezil in microspheres, entrapment efficiency, and amount of drug crystals present outside the microspheres

100 mg of microspheres prepared in each of examples 1 to 4 and comparative examples 1 to 3 was completely dissolved in acetonitrile and then diluted with a mobile phase. 20 uL of the diluted solution was injected into HPLC and measured at a detection wavelength of 318 nm.

In addition, 100 mg of microspheres prepared in each of examples 1 to 4 and comparative examples 1, 1-1 and 3 and washed with an aqueous ethanol solution in experimental example 2 was completely dissolved in acetonitrile and then diluted with a mobile phase. 20 uL of the diluted solution was injected into HPLC and measured at a detection wavelength of 318 nm.

Column: Luna phenyl-Hexyl, C18 5 um, 4.6 × 250 mm
Mobile phase: pH 2.0 tetrahydrofuran, 3:1 mixed solution of triethylamine solution (solution A) and methanol tetrahydrofuran solution (solution B)

Table 1 shows the measured drug entrapment efficiency (%) and the drug entrapment efficiency in microspheres after washing with ethanol.

[Table 1]

| | Theoretical drug loading amount (%) | Measured drug entrapment efficiency (%) (A) | Drug entrapment efficiency (%) after washing with EtOH (B) | Calculated amount (%) of drug crystals present outside microsphere (A-B) |
|---|---|---|---|---|
| Example 1 | 40% | 91.89 | 90.76 | 1.13 |
| Example 1-1 | 45% | 96.78 | 95.54 | 1.24 |
| Example 2 | 40% | 95.55 | 94.92 | 0.63 |
| Example 2-1 | 40% | 95.42 | 92.61 | 2.81 |
| Example 3 | 40% | 93.89 | 93.75 | 0.14 |
| Example 3-1 | 40% | 90.94 | 90.82 | 0.12 |
| Example 4 | 40% | 95.68 | 95.47 | 0.21 |
| Comparative example 1 | 40% | 92.71 | 90.39 | 2.32 |
| Comparative example 1-1 | 45% | 95.58 | 93.26 | 2.32 |
| Comparative example 2 | 40% | 93.90 | - | - |
| Comparative example 2-1 | 40% | 94.90 | - | - |
| Comparative example 3 | 40% | 95.97 | 93.22 | 2.75 |

<Explanation of terms used in the above table>

Theoretical drug loading amount (%) = Amount of drug introduced while preparing microspheres / (Amount of drug introduced while preparing microspheres + Amount of polymer introduced while preparing microspheres) $\times$ 100%

Measured drug entrapment efficiency (%) (A) = Measured amount (mg) of drug actually included in every 100 mg of microspheres immediately after preparation in comparative examples and examples / (100 mg of microspheres $\times$ theoretical drug loading amount (%)) $\times$ 100%

Drug entrapment efficiency after washing with ethanol (%) (B) = Measured amount (mg) of drug actually included in every 100 mg of microspheres after washing in experimental example 2 / (100 mg of microspheres $\times$ theoretical drug loading amount (%)) $\times$ 100%

$$\text{Calculated amount (\%) of drug crystals present outside microsphere} = A - B$$

As can be seen in comparative example 1 and comparative example 1-1, the amount of drug crystals present outside exceeded 2% in the microspheres prepared without applying a process of additional washing with ethanol or a surfactant.

However, in the case of the examples prepared by adding a salt to the continuous phase, the amount of drug crystals confirmed to be present outside the microspheres was very small.

Therefore, when microspheres are prepared by adding a salt such as NaCl to the continuous phase, the amount of drug crystals that may be present outside the microspheres may be minimized, and thus the actual entrapment efficiency of the drug in the microspheres may be maximized.

Experimental Example 4: Initial *in vitro* release test of microspheres

10 mg of the microspheres prepared in the examples and comparative examples were respectively added to 100 mL of a pH 7.4 HEPES solution and placed in a constant temperature shaking water bath maintained at 37.0°C. After 24 hours, the supernatant was taken and filtered with a 0.45 um syringe filter.

The initial daily dissolution amount of donepezil released from the microspheres was measured using HPLC. The column was XTerra Shield RP18 column 5 $\mu$m, 4.6 $\times$ 150 mm, the injection amount was 20 $\mu\ell$, the detection wavelength was 271 nm, and the mobile phase was pH 5.0 phosphate buffer solution and an acetonitrile solution (phosphate buffer solution: acetonitrile = 60: 40).

Table 2 below shows the dissolution rate for the 1st day of donepezil microspheres according to each example and comparative example.

[Table 2]

|  | Dissolution rate of the 1st day (%) |
|---|---|
| Example 1 | 3.25 |
| Example 1-1 | 7.64 |
| Example 2 | 6.06 |
| Example 2-1 | 11.72 |
| Example 3 | 5.25 |
| Example 3-1 | 3.26 |
| Example 4 | 10.25 |
| Comparative example 1 | 7.78 |
| Comparative example 1-1 | 10.87 |
| Comparative example 2 | 5.5 |
| Comparative example 2-1 | 4.9 |
| Comparative example 3 | 17.21 |

As can be seen in Table 2, example 1 (where NaCl was added in 1% to the continuous phase) and example 2 (where NaCl was added in 5% to the continuous phase) showed a relatively lower dissolution rate compared to comparative example 1 (where NaCl was not added to the continuous phase).

As in experimental example 3, the smaller the amount of drug crystals present outside the microspheres, the lower the dissolution rate of the 1st day.

As can be seen in example 3 and example 3-1, when KCl was used, it was confirmed that the same effect as that of the case of NaCl was exhibited.

In addition, even when the polymer in dispersed phase was PLGA (Resomer RG 753 H), the initial dissolution rate of microspheres in example 4 was lower than the initial dissolution rate of comparative example 3.

Experimental Example 5: Pharmacokinetic test of microspheres using SD rats

In order to confirm the effect of suppressing the initial release of microspheres prepared by adding salt to the continuous phase, the concentration of donepezil in the blood was measured after subcutaneous administration to the back of the neck of rats.

The microspheres prepared in example 1, comparative example 1, comparative example 2, and comparative example 2-1 were weighed so that the amount of donepezil administered in the microspheres per rat was 25.2 mg/kg, and then dispersed in 0.3 mL suspension and subcutaneously injected into SD rats.

At regular intervals, 0.3 mL of blood was collected from the jugular vein of the rat, kept in an ice-cooled state, and centrifuged to separate 100 uL of plasma. The separated plasma was analyzed for the concentration of donepezil using LC/MS/MS.

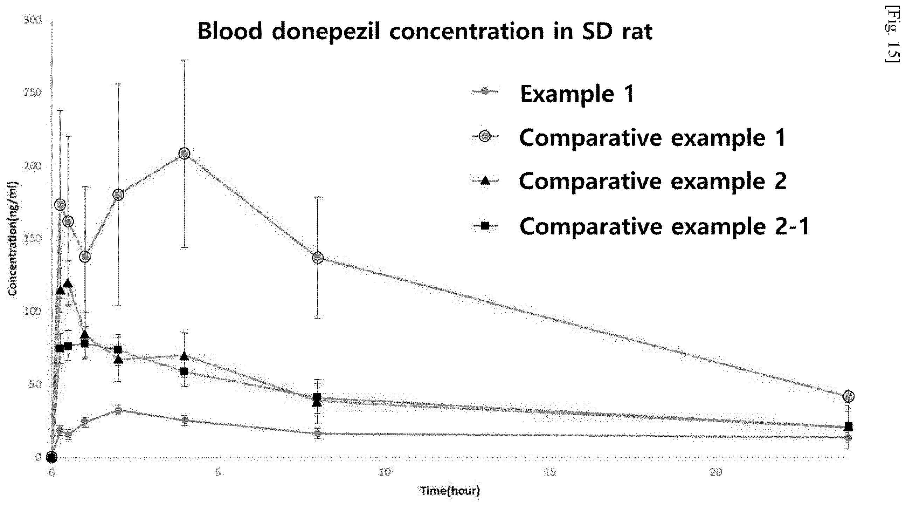

The measurement results are shown in Fig. 15.

As can be seen in Fig. 15, it may be confirmed that the microspheres of comparative example 1 to which a washing process with ethanol or a surfactant was not applied showed the highest Cmax of 220.5 ng/mL, whereas the microspheres of comparative example 2 washed with ethanol and the microspheres of comparative example 2-1 washed with a surfactant showed a lower Cmax of 141.03 ng/mL and 90.2 ng/mL, respectively, because drug crystals were removed by washing.

The microspheres of Example 1 prepared by adding NaCl in a content of 1% to the continuous phase showed the lowest Cmax of 58.9 ng/mL because drug crystals around the microspheres were removed and surface pores were also removed. In other words, it was possible to prevent the release of an excessive amount of drug in the initial stage. This result was similar to the *in vitro* result.

When microspheres are prepared by adding NaCl to the continuous phase according to the present invention, the formation of drug crystals around the microspheres is suppressed, and thus a separate process of removing drug crystals may not be additionally introduced and also the drug entrapment efficiency of the microspheres may be improved. In addition, drug crystals are not formed around the microspheres, and thus initial drug release may be suppressed when the microspheres are injected into the body.

## Industrial applicability

According to the present invention, drug crystals are not formed around the microspheres, and thus initial drug release may be suppressed when the microspheres are injected into the body. In addition, the drug is released continuously, and thus the medicinal effect may be exhibited for a fairly long time with a single injection.

## Claims

1. A method for preparing a microsphere, comprising:

   dissolving an active ingredient and a biodegradable polymer in an organic solvent to prepare a dispersed phase;
   dissolving a salt in water to prepare a continuous phase;
   mixing and stirring the dispersed phase and the continuous phase to form an emulsion;
   removing the organic solvent; and
   drying.

2. The method of claim 1, wherein the salt is at least one selected from the group consisting of sodium chloride (NaCl), potassium chloride (KCl), calcium chloride (CaCl$_2$), magnesium sulfate (MgSO4), sodium sulfate (Na2SO4), mannitol, ammonium, potassium sulfate, disodium phosphate, dipotassium phosphate, trisodium phosphate, disodium citrate, trisodium citrate and sodium succinate.

3. The method of claim 2, wherein the salt has a concentration of 1 to 10% (w/v) in the continuous phase.

4. The method of claim 1, wherein the biodegradable polymer is at least one selected from the group consisting of polylactide, poly(lactide-co-glycolide), polyglycolactide and poly(lactide-co-glycolide)glucose.

5. The method of claim 1, wherein the continuous phase further comprises a water-soluble polymer.

6. The method of claim 5, wherein the water-soluble polymer is at least one selected from the group consisting of polyvinyl alcohol, polysorbate, poloxamer, polyvinylpyrrolidone, polyvinylmethyl ether and polyvinyl ether.

7. The method of claim 1, wherein the active ingredient is a substance acting on the central nervous system.

8. The method of claim 7, wherein the active ingredient is a substance having therapeutic activity against dementia or therapeutic activity against Alzheimer's disease.

9. A microsphere prepared according to the method of any one of claims 1 to 8.

10. A sustained release injection comprising the microsphere according to claim 9.

[Fig. 1]

[Fig. 2]

[Fig. 3]

[Fig. 4]

[Fig. 5]

[Fig. 6]

[Fig. 7]

[Fig. 8]

[Fig. 9]

[Fig. 10]

[Fig. 11]

[Fig. 12]

[Fig. 13]

[Fig. 14]

[Fig. 15]

Blood donepezil concentration in SD rat

EP 4 056 170 A1

<table>
<tr><td colspan="2" align="center">**INTERNATIONAL SEARCH REPORT**</td><td colspan="2">International application No.<br>**PCT/KR2020/015565**</td></tr>
</table>

**A.    CLASSIFICATION OF SUBJECT MATTER**

**A61K 9/16**(2006.01)i; **A61K 9/00**(2006.01)i; **A61P 25/28**(2006.01)i

According to International Patent Classification (IPC) or to both national classification and IPC

**B.    FIELDS SEARCHED**

Minimum documentation searched (classification system followed by classification symbols)

A61K 9/16(2006.01); A61J 3/02(2006.01); A61K 47/34(2006.01); A61K 47/40(2006.01); A61K 9/22(2006.01); A61K 9/50(2006.01); A61K 9/52(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 마이크로스피어(microsphere), 염화나트륨(NaCl), 고분자(polymer), 주사제(injections), 서방성(sustained release)

**C.    DOCUMENTS CONSIDERED TO BE RELEVANT**

| Category* | Citation of document, with indication, where appropriate, of the relevant passages | Relevant to claim No. |
|---|---|---|
| Y | KR 10-2004-0066191 A (TAKEDA PHARMACEUTICAL COMPANY LIMITED) 23 July 2004 (2004-07-23)<br>    See claims 1-18 and 21-25; and page 13. | 1-10 |
| Y | KR 10-2008-0004075 A (AMOREPACIFIC CORPORATION) 09 January 2008 (2008-01-09)<br>    See claims 1-18; and paragraphs [0029], [0040] and [0049]-[0056]. | 1-10 |
| A | KR 10-2018-0131077 A (DAEWOONG PHARMACEUTICAL CO., LTD.) 10 December 2018 (2018-12-10)<br>    See claims 1-16. | 1-10 |
| A | KR 10-0681213 B1 (TANABE SEIYAKU CO., LTD.) 09 February 2007 (2007-02-09)<br>    See entire document. | 1-10 |
| A | KR 10-2004-0066548 A (KOLON PHARMACEUTICAL CO., LTD.) 27 July 2004 (2004-07-27)<br>    See entire document. | 1-10 |

☐ Further documents are listed in the continuation of Box C.          ☑ See patent family annex.

| | |
|---|---|
| *    Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **24 February 2021** | **24 February 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

INTERNATIONAL SEARCH REPORT
Information on patent family members

International application No.

PCT/KR2020/015565

| Patent document cited in search report | | | Publication date (day/month/year) | Patent family member(s) | | | Publication date (day/month/year) |
|---|---|---|---|---|---|---|---|
| KR | 10-2004-0066191 | A | 23 July 2004 | AU | 2002-367105 | A1 | 15 July 2003 |
| | | | | CA | 2471521 | A1 | 10 July 2003 |
| | | | | CA | 2471521 | C | 02 November 2010 |
| | | | | CN | 101444476 | A | 03 June 2009 |
| | | | | CN | 101444476 | B | 30 November 2011 |
| | | | | CN | 1620285 | A | 25 May 2005 |
| | | | | EP | 1466596 | A1 | 13 October 2004 |
| | | | | EP | 1466596 | B1 | 31 August 2016 |
| | | | | JP | 2003-252751 | A | 10 September 2003 |
| | | | | KR | 10-0994658 | B1 | 16 November 2010 |
| | | | | US | 2005-0064039 | A1 | 24 March 2005 |
| | | | | WO | 03-055470 | A1 | 10 July 2003 |
| KR | 10-2008-0004075 | A | 09 January 2008 | KR | 10-0908517 | B1 | 20 July 2009 |
| KR | 10-2018-0131077 | A | 10 December 2018 | CN | 110944627 | A | 31 March 2020 |
| | | | | JP | 2020-522505 | A | 30 July 2020 |
| | | | | KR | 10-2142026 | B1 | 06 August 2020 |
| | | | | US | 2020-0113836 | A1 | 16 April 2020 |
| | | | | WO | 2018-221884 | A1 | 06 December 2018 |
| KR | 10-0681213 | B1 | 09 February 2007 | AU | 2003-262048 | A1 | 30 April 2004 |
| | | | | CA | 2497723 | A1 | 25 March 2004 |
| | | | | CN | 101229098 | A | 30 July 2008 |
| | | | | CN | 101229098 | B | 29 February 2012 |
| | | | | CN | 1688275 | A | 26 October 2005 |
| | | | | CN | 1688275 | B | 30 May 2012 |
| | | | | EP | 1537846 | A1 | 08 June 2005 |
| | | | | JP | 4690040 | B2 | 01 June 2011 |
| | | | | KR | 10-2005-0042808 | A | 10 May 2005 |
| | | | | US | 2005-0271731 | A1 | 08 December 2005 |
| | | | | US | 2007-0182040 | A1 | 09 August 2007 |
| | | | | WO | 2004-024056 | A1 | 25 March 2004 |
| KR | 10-2004-0066548 | A | 27 July 2004 | None | | | |

Form PCT/ISA/210 (patent family annex) (July 2019)